## Europäisches Patentamt

## European Patent Office

## Office européen des brevets

(11) Veröffentlichungsnummer: **0 057 404**
**A1**

# EUROPÄISCHE PATENTANMELDUNG

(21) Anmeldenummer: 82100501.4

(22) Anmeldetag: **26.01.82**

(51) Int. Cl.³: **B 41 F 33/00**, G 01 N 33/32

(30) Priorität: 30.01.81 DE 3103088

(43) Veröffentlichungstag der Anmeldung: **11.08.82**
**Patentblatt 82/32**

(84) Benannte Vertragsstaaten: **CH DE FR GB IT LI NL**

(71) Anmelder: **Forschungsgesellschaft Druckmaschinen e.V., Postfach 71 01 09,**
**D-6000 Frankfurt/Main-Niederrad 71 (DE)**

(72) Erfinder: **Rodriquez-Giles, Jorge, Dr.rer.nat.,**
**August-Victoria-Strasse 6, D-6200 Wiesbaden (DE)**
Erfinder: **Spiegel, Nikolaus, Dipl.-Ing., Zeilharder**
**Strasse 14a, D-6107 Reinheim 4 (DE)**
Erfinder: **Scheuter, K.R., Prof. Dipl.-Ing.,**
**Schillerstrasse 9, D-6100 Darmstadt-Eberstadt (DE)**

(74) Vertreter: **Munk, Ludwig, Dipl.-Ing.,**
**Prinzregentenstrasse 1, D-8900 Augsburg (DE)**

(54) **Vorrichtung zur Bestimmung der Abriebfestigkeit von schichtförmig auf einen zugeordneten Träger aufgebrachten Medien.**

(57) Um bei einer Vorrichtung zur Bestimmung der Abriebfestigkeit eines einen geeigneten Träger (1) benetzenden Mediums mit einem durchsichtigen, mit definierter Kraft auf den Träger (1) aufpressbaren Abschmierer (4) eine verzögerungsfreie und vom Untergrund unabhängige und daher universell verwendbare Messwertbildung zu gewährleisten, wird zum Nachweis der Benetzung der Kontaktzone (6) des Abschmierers (4) die Extinktion der Totalreflexion verwendet.

## Vorrichtung zur Bestimmung der Abriebfestigkeit von schichtförmig auf einen zugeordneten Träger aufgebrachten Medien

Die Erfindung betrifft eine Vorrichtung zur Bestimmung der Abriebfestigkeit von auf einen Träger aufgebrachten Druckfarben, mit einem mit einstellbarer Kraft auf den von einer Gegenkraft, vorzugsweise von einem hiervon leicht umschlungenen Stahlteil, wie einer Stahlwalze, gestützten Träger andrückbaren Abschmierer aus optisch durchsichtigem Material, der einer auf die Berührungsstelle zwischen Träger und Abschmierer fokusierten Beleuchtungseinrichtung ausgesetzt ist und dem eine im Bereich des zurückgeworfenen, aus ihm austretenden Lichts angeordnete Beobachtungseinrichtung zugeordnet ist.

Eine Vorrichtung dieser Art ist aus der DE-AS 29 17 519 bekannt. Bei dieser bekannten Anordnung wird praktisch der Träger, im konkreten Fall eine Papierbahn, beobachtet und die Intensität des vom Träger durch den durchsichtigen Abschmierer hindurch zurückgestrahlten Lichts gemessen. Die Anwesenheit von abschmierenden Medien, im konkreten Fall noch nicht trockener, d. h. nicht abriebfester Druckfarbe, auf der Kontaktfläche des Abschmierers setzt deren Transparenz herab, so daß dementsprechend auch die Intensität des vom Träger zurückstrahlbaren Lichts abnimmt. Die Änderung der Transparenz der Kontaktfläche dient bei der bekannten Anordnung als Maß für den Trockengrad bzw. die Abriebfestigkeit.

Die bei der bekannten Anordnung verwendete Transparenzmessung verlangt jedoch einen sauberen, reflektierenden Hintergrund. Dies ist bei der Verarbeitung von weißem Papier gegeben, bei der Verarbeitung von Buntpapier jedoch nicht, was die Verwendbarkeit der bekannten Anordnung ganz entscheidend einschränkt. Ganz abgesehen davon ist auch bei der Verarbeitung von weißem als Träger dienendem Papier nur dort ein reflektierender Hintergrund vorhanden, wo das Papier nicht bedruckt ist. Das ist in der Regel nur im Bereich des sogenannten Kanalstreifens, der pro Zylinderumfang im allgemeinen nur einmal vorhanden ist., der Fall. Dies führt daher zu einer nicht unbeträchtlichen Verzögerung des Meßergebnisses. Außerdem sind hierbei Steuermarken oder Synchronimpulese erforderlich, welche den Durchgang jedes Spannkanals durch die Kontaktzone signalisieren. Außerdem ist es hierbei erforderlich, daß die Kontaktfläche des Abschmierers beim Durchgang des schmalen, als Meßzone dienenden Spannkanalstreifens wieder so weit gesäubert

- 3 -

wird, daß die ursprüngliche Transparenz vorhanden ist.
Es ist daher erforderlich, den Abschmierer mit verhältnismäßig großer Kraft an den Träger anzudrücken.
Dies kann jedoch nicht nur einen verhältnismäßig starken Verschleiß im Bereich der Kontaktzone bewirken,
sondern ergibt bei fortschreitendem Verschleiß auch
eine nicht unbeträchtliche Vergrößerung der Kontaktzone. Unter Umständen ist es hier notwendig für die
Säuberung zwei nacheinanderfolgende Spannkanaldurchgänge zu benutzen. Bei vielen Verfahren, wie z. B.
beim Tiefdruck, wird aber oft die gesamte Trägeroberfläche beschichtet, so daß hierbei das bekannte Meßverfahren nicht anwendbar ist.

Die Erfahrung hat ferner gezeigt, daß die bekannte Anordnung nicht ohne sogenannte Referenzspur auskommt,
die auf sauberem, reflektierendem Untergrund geführt
wird, um durch Vergleich die unterschiedliche Lichtrückstreuung des Untergrunds zu kompensieren. Trotzdem zwingen örtliche Abweichungen der Lichtrückstreuung des Untergrunds zur Mittelwertbildung über mehrere Messungen,
was zu einer weiteren Verzögerung der Meßergebnisse
führt.

Hiervon ausgehend ist es daher die Aufgabe der vorliegenden Erfindung unter Vermeidung der Nachteile und
Beibehaltung der Vorteile der bekannten Anordnung eine
Vorrichtung eingangs erwähnter Art zu schaffen, welche
die Meßwerte nicht nur verzögerungsfrei, sondern auch
unabhängig von der Qualität und dem Tragbild des Trägers liefert und die gleichzeitig mit einer vergleichsweise geringen Anpreßkraft auskommt und zudem universell
einsetzbar ist.

Die Lösung dieser Aufgabe gelingt gemäß der Erfindung in überraschend einfacher Weise dadurch, daß die optische Achse der Beleuchtungseinrichtung gegenüber einer zu der die Kontaktzone zwischen Träger und Abschmierer enthaltenden Tangentialfläche lotrechten Ebene um jeweils einen Winkel geneigt ist, der zumindest gleich oder vorzugsweise größer als der Grenzwinkel der Totalreflexion für das den Abschmierer bildende Material ist.

Mit diesen Maßnahmen ist es in vorteilhafter Weise möglich, eine infolge mangelnder Abriebfestigkeit des auf dem Träger enthaltenen Mediums über die Extinktion der Totalreflexion nachzuweisen, Das auf unbenetzte Stellen des Abschmierers auftreffende Licht wird hierbei infolge der Totalreflexion wie von einer Spiegelfläche reflektiert. An benetzten Stellen des Berührungsbereichs ist die Totalreflexion gestört. Diese Stellen erscheinen von der Beobachtungseinrichtung aus gesehen, deren optische Achse zweckmäßig spiegelbildlich zur optischen Achse der Beleuchtungseinrichtung verläuft, d. h. entgegengesetzt zu dieser um denselben Winkel gegenüber der zur Tangentialebene lotrechten Ebene geneigt ist, praktisch als dunkle Flecken im Farbton des Mediums, wodurch die Intensität des reflektierten Lichts geschwächt wird, was als Maß für die Abriebfestigkeit verwendet werden kann. Da das total reflektierte Licht den Abschmierer nicht verläßt, sondern von der den Berührungsbereich enthaltenden Grenzschicht reflektiert wird, spielt der durch den Träger gebildete Hintergrund hier in vorteilhafter Weise keine Rolle mehr. Die erfindungsgemäße Anordnung kommt daher in vorteilhafter Weise auch ohne schichtfreie Meßzone aus, was nicht nur eine universelle Verwendbarkeit gewährleistet, sondern auch eine verzögerungsfreie, totzeitlose Bereitstellung der gewünschten Meßwerte sicherstellt. Außerdem gewährleisten die erfindungsgemäßen Maßnahmen in vorteilhafter Weise eine hohe Meßempfindlichkeit, da der Berührungsbereich verhältnismäßig klein sein kann, so daß das infolge mangelnder Abriebfestigkeit hierauf übertragene Medium in verhältnismäßig hoher Konzentration vorliegt. Hierdurch ist es möglich, den Anpreßdruck zwischen Abschmierer und Träger in vorteilhafter Weise bis auf einen Schwellenwert zurückzunehmen, so daß eine für das Auge sichtbare Beschädigung der Anordnung des auf dem Träger enthaltenen, aushärtenden Mediums, wie z. B. das auf einer Papierbahn enthaltene Druckbild, auf jeden Fall unterbleibt, was eine Messung der Abriebfestigkeit während des laufenden Betriebs ermöglicht. Außerdem ist hierdurch sichergestellt, daß der im Berührungsbereich sich ergebende Verschleiß in Grenzen bleibt. Ein Aufbau des nicht abriebfesten Mediums in der Berührungszone des Abschmierers bleibt aus, da durch die Relativbewegung zwischen Träger und Abschmierer ein ständiger Austausch des Mediums stattfindet. Die völlige Unabhängigkeit der hier möglichen Messung der Abriebfestigkeit vom Erscheinungsbild des aushärtenden Mediums auf dem Träger ermöglich in vorteilhafter Weise auch eine beliebige Einstellung des Abschmierers über der Trägerbreite, wodurch sich in vorteilhafter Weise besonders gefährdete Bereich erfassen lassen, was die Aussagekraft des erzielbaren Meßergebnisses erhöht.

Gemäß einer besonders zu bevorzugenden Ausgestaltung der übergeordneten Maßnahmen kann der Abschmierer die Form eines totalreflektierenden Prismas aufweisen, das gegenüber dem bewegten Träger festgehalten ist und diesen im Bereich seiner Hypotenusenfläche berührt. Diese Maßnahmen ergeben in vorteilhafter Weise eine einfache und kompakte Ausführung, die sich als besonders wirtschaftlich erweist. In zweckmäßiger Weise kann dabei der Abschmierer eine den Berührungsbereich enthaltende, vorzugsweise durch optischen Kitt mit dem übrigen Material verbundene Schutzplatte aus verschleißfestem, praktisch denselben Brechungsindex wie das übrige Material aufweisendem Material, vorzugsweise aus synthetischem Saphir, aufweisen. Diese Maßnahme gewährleistet in vorteilhafter Weise eine konstante Berührungsgeometrie über lange Betriebszeiten hinweg, was sich vorteilhaft auf die Meßgenauigkeit auswirkt.

Eine weitere besonders zu bevorzugende Fortbildung der übergeordneten Maßnahmen kann darin bestehen, daß jeder Eingang der Beobachtungseinrichtung mit einer Doppelschlitzblende versehen ist. Hiermit ist es möglich, Streulicht auszuschalten, so daß die Beobachtungseinrichtung ausschließlich mit parallelem Licht versorgt wird, was eine genaue Abtastung der Berührungsstelle ermöglicht. Zweckmäßig kann dabei zumindest der eingangsseitige Schlitz der Doppelschlitzblende einstellbar sein, wodurch in jedem Einzelfall eine hohe Genauigkeit erreichbar ist.

In weiterer Fortbildung der übergeordneten Maßnahmen kann der Doppelschlitzblende jeweils mindestens ein Filter nachgeordnet sein. Hierdurch ist es in vorteilhafter Weise möglich, das Härtungsverhalten einzelner Komponenten des auf dem Träger enthaltenen, aushärtenden Mediums, wie beispielsweise das Verhalten einzelner Farbkomponenten eines auf einem Bedruckstoff vorhandenen Druckbilds, zu bestimmen.

Aus der DE-AS 29 17 519 ist es zwar ferner bekannt, daß der Abschmierer ein Meßfeld und ein Referenzfeld aufweist, denen im Bereich der Beobachtungseinrichtung jeweils ein Fotoempfänger zugeordnet ist. Eine derartige Referenzmessung ist bei der bekannten Anordnung zur Erfassung der Höhe der Lichtrückstreuung der den reflektierenden Hintergrund bildenden Papierbahn unbedingt erforderlich und ermöglicht bei der erfindungsgemäßen Anordnung die Ausschaltung von Schwankungen im Bereich der Beleuchtungseinrichtung. Bei der bekannten Anordnung ist es jedoch unbedingt erforderlich, das Referenzfeld einer schichtfreien Trägeroberfläche zuzuordnen. Die bekannte Anordnung erweist sich daher hinsichtlich einer vielfach erwünschten Anpassung der Lage des Abschmierers an die individuellen Verhältnisse eines Druckbilds als nicht variabel genug. Die erfindungsgemäßen Maßnahmen benötigen kein schichtfreies Referenzfeld auf dem Träger. Als besonders vorteilhaft kann es sich dabei erweisen, wenn die dem Träger zugewandte Seite des Abschmierers im Bereich des Referenzfelds als vom Träger abgewandte Spiegelfläche ausgebildet ist, was eine direkte Reflexion der auftreffenden Lichtstrahlen bewirkt und eine völlige Unabhängigkeit von der Beschaffenheit des Hintergrunds, d. h. des Trägers und des hierauf enthaltenen aushärtenden Mediums gewährleistet.

In weiterer Fortbildung der Erfindung kann den Fotoempfängern der Beobachtungseinrichtung eine Differenzbildungseinrichtung nachgeordnet sein. Die hiervon gelieferte Differenz der Meßergebnisse aus dem Referenzfeld und dem Meßfeld ist ein direktes Maß für die Störung der Totalreflexion im Bereich des Meßfelds, die selbst ein Maß für die Abriebfestigkeit darstellt. Bezogen auf die gewählte Anpreßkraft ist das Medium abriebfest, d. h. ausgehärtet, wenn die Differenz null ist. Zweckmäßig kann dabei der Differenzbildungseinrichtung eine Speichereinrichtung für Spitzenwerte nachgeordnet sein, deren Ausgang einer Regeleinrichtung eingebbar ist, mittels welcher ein dem Abschmierer vorgeordneter Trockner direkt oder indirekt regelbar ist. Eine derartige Einrichtung trägt der stochastischen Natur des Abschmiervorgangs Rechnung und ergibt in vorteilhafter Weise eine hohe Stabilität der gesamten Regelstrecke. Zweckmäßig kann dabei die auf den Abschmierer wirkende Anpreßkraft mit Hilfe der Regeleinrichtung so verstellbar sein, daß eine gleichbleibende Differenz der Meßergebnisse aus dem Referenzfeld und dem Meßfeld aufrechterhalten wird, wobei die Differenz vorteilhafterweise so gewählt wird, daß sie gerade keiner sichtbaren Verschmierung entspricht, jedoch reproduzierbar gemessen werden kann. In diesem Fall wird die Anpreßkraft der Regeleinrichtung des Trockners eingegeben. Hierbei ist es in vorteilhafter Weise möglich, ganz sicher zu verhindern, daß der Abschmierer die Anordnung des trocknenden Mediums, etwa das auf einer Papierbahn sich befindende Druckbild so weit beschädigt, daß es mit normalem Auge erkennbar ist.

Eine weitere vorteilhafte Fortbildung der übergeordneten Maßnahmen kann darin bestehen, daß der bzw. die Fotoempfänger durch mindestens eine gehäuseseitig abgestützte Moosgummiplatte hinterlegt ist bzw. sind, die eine umlaufende, auf der Unterlage des bzw.

der Fotoempfänger lichtdicht aufliegende Dichtkante aufweist. Hierdurch ist sichergestellt, daß kein Fremdlicht eindringen kann.

In weiterer zweckmäßiger Fortbildung der übergeordneten Maßnahmen kann der Abschmierer in an sich bekannter Weise an einem Schwenkarm befestigt sein, der zweckmäßig in Achsrichtung verstellbar auf einem Schlitten gelagert ist, der über der Breite des Trägers verstellbar ist. Die axiale Verstellbarkeit des Schwenkarms ermöglicht in vorteilhafter Weise eine genaue Justierung der Kontaktstelle.

Weitere vorteilhafte Ausgestaltungen und zweckmäßige Weiterbildungen der übergeordneten Maßnahmen ergeben sich aus der nachstehenden Beschreibung einiger Ausführungsbeispiele anhand der Zeichnung in Verbindung mit den restlichen Unteransprüchen. Hierbei zeigen:

Figur 1     eine Seitenansicht einer ersten Ausführungsform anhand einer Vorrichtung zur Bestimmung der Abriebfestigkeit von Druckfarben auf einer bedruckten Papierbahn,

Figur 2     eine mit einem Blockschaltbild verknüpfte, schematische Draufsicht auf die Beobachtungsseite der Anordnung nach Figur 1 und

Figuren     weitere Ausführungsbeispiele der Erfindung.
3 und 4

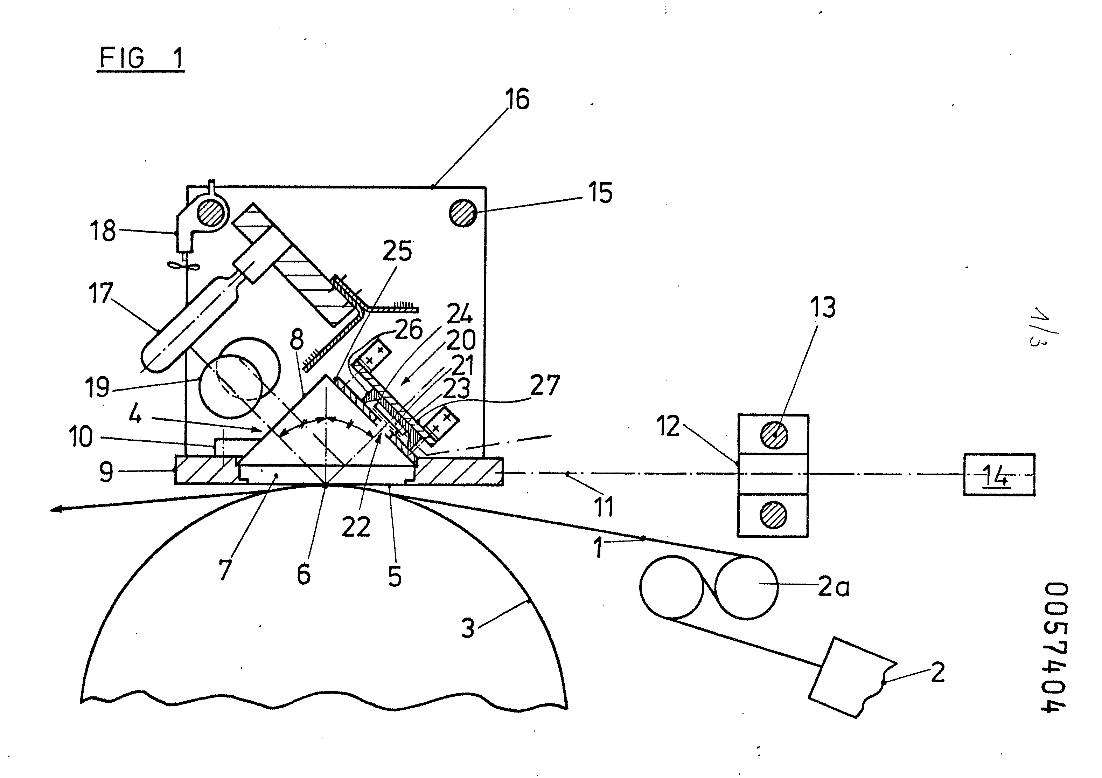

Die in Figur 1 bei 1 angedeutete, als Träger dienende Papierbahn soll mit Druckfarbe bedruckt sein. Die das hier nicht dargestellte Druckwerk verlassende Papierbahn durchläuft in der Regel einen dem Druckwerk nachgeordneten, hier bei 2 angedeuteten Trockner dem eine oder mehrere Kühlwalzen der bei 2a angedeuteten Art nachgeordnet sein können. Anschließend kann die Papierbahn über Leitwalzen 3 oder dergleichen einem nicht dargestellten Falzapparat zugeführt und dort zu Falzprodukten weiterverarbeitet werden. Der Aufbau und die Wirkungsweise der vorstehend genannten Einrichtungen sind an sich bekannt und bedürfen daher keiner näheren Erläuterung mehr.

Eine störungsfreie Weiterverarbeitung der Papierbahn ist nur dann möglich, wenn die hierauf sich befindende Druckfarbe eine ausreichende Abriebfestigkeit erreicht hat. Die modernen Druckfarben bilden zwar verhältnismäßig schnell eine geschlossene Oberfläche, die sich oft als ausreichend ablegefest erweisen kann. Eine störungsfreie Weiterverarbeitung etwa in einem Falzapparat oder einer Verpackungsmaschine oder dergleichen erfordert jedoch eine abriebfeste Oberfläche. Es erweist sich daher vielfach als sehr erwünscht, die Abriebfestigkeit des Druckbilds zu bestimmen. Die hierfür vorgesehene Vorrichtung besteht aus einem Abschmierer 4, im dargestellten Ausführungsbeispiel in Form eines totalreflektierenden Glasprismas, das mit seiner Hypotenuse 5 an die Papierbahn 1 andrückbar ist, die im Andrückbereich durch eine entsprechende Gegenkraft, erzeugt durch einen Prallstrahl, eine Wendestange, Leitwalze, die letzte Kühlwalze oder dergleichen, gestützt ist. Im dargestellten Ausführungsbeispiel liegt der Abschmierer 4 im Bereich des höchsten Punkts der von der Papierbahn 1 leicht umschlungenen Leitwalze 3 auf dieser auf. Die Leitwalze 3 soll hier einen Stahlmantel besitzen, so daß sich praktisch keine nennenswerte Abflachung ergibt, was in vorteilhafter Weise eine exakte Begrenzung der mit 6 bezeichneten Kontaktfläche ermöglicht. Das den Abschmierer 4 bildende Glasprisma ist im dargestellten Ausführungsbeispiel hypotenusenseitig durch eine Schutzplatte 7 aus synthetischem Saphir verstärkt. Dieses Material erweist sich als äußerst verschleißfest und besitzt praktisch denselben Brechungsindex wie Glas. Zur Verbindung der Schutzplatte 7 mit dem Glaskörper 8 des als Ganzes mit 4 bezeichneten Abschmierers kann einfach optischer Kitt, etwa in Form von sogenanntem Kanada-Balsam, oder optisches Öl, Verwendung finden. Die Schutzplatte 7 kann im Bereich der Kontaktzone 6 einen leichten, der Rundung der hier die Stütze bildenden Leitwalze 3 entsprechenden Einschliff aufweisen. Im dargestellten Ausführungsbeispiel ist die Schutzplatte 7 mit einer ebenen Auflagefläche versehen, was einen besonders schmalen Kontaktbereich und damit im Falle des Abschmierens von Farbe eine hohe Farbkonzentration im Kontaktbereich ergibt. Wie Versuche gezeigt haben, verteilt sich hierbei die Farbe auf ein Feld von höchstens 2 mm Breite. Die Ausdehnung des den Abschmierer 4 bildenden Prismas senkrecht zur Zeichenebene kann etwa 25 - 50 mm betragen. Sofern die auf der unter dem Abschmierer 4 hindurchgehenden Papierbahn 1 sich befindende Farbe abriebfest ist, ergibt sich im Bereich der Kontaktzone 6 der Hypotenuse 5 des Abschmierers keine Verschmutzung. Eine weniger abriebfeste Farbe schmiert ab, d. h. verschmutzt die Hypotenusenfläche 5 im Bereich der Kontaktzone 6. Die Kraft, mit welcher der Abschmierer 4 an die Papierbahn 1 angedrückt wird, ist dabei zweckmäßig so eingestellt, daß die durch den Abschmierer 4 bewirkte Verwischung des Druckbilds mit bloßem Auge nicht erkennbar ist, was den Anfall von Makulatur vermeidet und dennoch eine Überwachung der Abriebfestigkeit während des laufenden Betriebs ermöglicht.

Das den Abschmierer 4 bildende Prisma ist in einen Halterahmen 9 eingelassen und durch bei 10 angedeutete Spannorgane gesichert. Der Halterahmen 9 ist an einem hier lediglich durch seine Mittellinie angedeuteten Schwenkarm 11 befestigt, der mit seiner Schwenkachse auf einem Schlitten 12 gelagert ist, der seinerseits auf Traversen 13 quer zur Laufrichtung der Papierbahn 1 über deren Breite verstellbar ist. Zur Bewerkstelligung einer genauen Justierbarkeit der Kontaktzone 6 ist der Schwenkarm 11 zweckmäßig in axialer Richtung einstellbar. Dies läßt sich etwa mit Hilfe einer die Schwenkachse enthaltenden, vom Schwenkarm 11 durchsetzten Spannmuffe bewerkstelligen. Die im Bereich der Kontaktzone wirksame Anpreßkraft wird im dargestellten Ausführungsbeispiel durch Verwendung eines Gegengewichts 14 eingestellt. Hierzu ist der Schwenkhebel 11 als zweiarmiger Hebel ausgebildet, der einerseits den Halterahmen 9 und andererseits das Gegengewicht 14 aufnimmt. Das Gegengewicht 14 ist zur Regulierung der Anpreßkraft vorteilhaft in Achsrichtung verstellbar angeordnet. Eine Anpreßkraft in der Größenordnung von etwa 2 N hat sich bei einer Gesamtbreite des Abschmierers von 50 mm für die Bestimmung der Abriebfestigkeit von Druckfarbe als besonders vorteilhaft erwiesen. Hierbei unterbleibt einerseits eine mit bloßem Auge erkennbare Beschädigung des Druckbilds und ist andererseits eine Selbstreinigung der Kontaktzone 6 innerhalb verhältnismäßig kurzer Zeit gewährleistet. Am Halterahmen 9 sind durch Traversen 15 miteinander verbundene Seitenwandungen 16 befestigt, die zur Halterung weiterer dem

0057404

Abschmierer 4 zugeordneter Aggregate dienen.

Eine Kathetenfläche des den Abschmierer 4 bildenden Prismas wird von einer Beleuchtungseinrichtung 17, beispielsweise einer Halogenlampe, beleuchtet. Der Lampe der Beleuchtungseinrichtung 17 ist im dargestellten Ausführungsbeispiel eine durch einen Ventilator 18 gebildete Kühleinrichtung zugeordnet, was eine hohe Lebensdauer gewährleistet. Die Beleuchtungseinrichtung 17 ist auf die Kontaktzone 6 fokusiert. Hierzu ist eine Linse 19, im dargestellten Ausführungsbeispiel einfach eine Zylinderlinse, vorgesehen. Die Beleuchtungseinrichtung 17 ist gegenüber dem Abschmierer 4 so angeordnet, daß die optische Achse des von der Beleuchtungseinrichtung 17 ausgehenden Lichts gegenüber einer auf der die Kontaktzone 6 enthaltenden Hypotenusenfläche 5 lotrecht stehenden Ebene um einen Winkel $\alpha$ geneigt, ist, der größer als der Grenzwinkel der Totalreflexion für das den Abschmierer 4 bildende Material ist. Dies ist bei einem totalreflektierenden Prisma etwa dann der Fall, wenn die optische Achse der Beleuchtungseinrichtung die zugewandte Kathetenfläche lotrecht durchstößt. Infolge der an der Hypogenusenfläche 5 stattfindenden Totalreflexion tritt das auf die Kontaktzone 6 fokusierte Licht hier nicht im Bereich dieser Kontaktzone aus dem Prisma aus, sondern wird wie bei Verwendung eines Spiegels reflektiert und tritt dementsprechend durch die der Beleuchtungseinrichtung 17 abgewandte Kathetenfläche aus dem Prisma aus. Die Neigung der optischen Achse des Austrittsstrahls entspricht dem Spiegelbild der Neigung der optischen Achse des Eintrittsstrahls. Der aus dem Abschmierer 4 austretende Strahl wird mit Hilfe einer der Beleuchtungseinrichtung 17 gegenüberliegenden, als Ganzes mit 20 bezeichneten Beobachtungs-

0057404

einrichtung erfaßt.

Solange die Hypotenusenfläche 5 im Bereich der Kontaktzone 6 sauber ist, wird das auf die Kontaktzone 6 einfallende Licht mit voller Intensität reflektiert. Sobald im Bereich der Kontaktzone 6 optischer Kontakt mit anderem Material besteht, d. h. sobald der Abstand dieses anderen Materials zur Unterseite der die Kontaktzone 6 enthaltenden Hypotenusenfläche 5 kleiner als die Wellenlänge des Lichts ist, wovon beim Abschmieren von Farbe, also bei einer Benetzung mit noch nicht vollkommen trockener Farbe auszugehen ist, ist die Totalreflexion gestört. Die Stellen mit Farbkontakt erscheinen von der Beobachtungseinrichtung 20 aus gesehen als dunkle Flecken im Farbton des Mediums Druckfarbe, weil an diesen Stellen keine Reflexion stattfindet. Die Intensität des reflektierten Lichts ist daher in einem solchen Falle schwächer als bei vollkommen sauberer Kontaktzone. Die in der Abnahme der Lichtintensität sich äußernde Extinktion der Totalreflexion ist von der Abriebfestigkeit der auf der Papierbahn 1 sich befindenden Druckfarbe eindeutig abhängig, d. h. bei geringer Abriebfestigkeit erfolgt eine starke Abnahme der Intensität des reflektierten Lichts und umgekehrt.

Die Beobachtungseinrichtung 20 besteht aus einem Fotoempfänger 21, der bei Beaufschlagung mit Licht einen Strom von der Lichtintensität entsprechender Stärke abgibt. Zur Bewerkstelligung einer genauen Abtastung der Kontaktzone 6 mittels des Fotoempfängers 21 ist diesem eine Doppelschlitzblende 22 vorgeordnet, die einfach durch voneinander distanzierte Platinen gebildet werden kann. Im Zwischenraum zwischen den voneinander distanzierten Platinen fängt sich sogenanntes Streulicht, so daß vorteilhaft nur paralleles Licht zur Wirkung kommt. Der eingangsseitige
Schlitz der Doppelschlitzblende 22 kann vorteilhaft
einstellbar ausgebildet sein, was eine genaue Justierung ermöglicht. Im dargestellten Ausführungsbeispiel ist zwischen Doppelschlitzblende 22 und
Fotoempfänger 21 ein durch eine gestrichelte Linie
angedeutetes Filter 23 angeordnet, das bestimmten
Farbkomponenten zugeordnet sein kann. Die Doppelschlitzblende 22 liegt direkt auf der ausgangsseitigen Kathetenfläche des den Abschmierer 4 bildenden Prismas auf. Der Fotoempfänger 21 und evtl. das
diesem vorgeordnete Filter 23 sind hierauf mittels
einer Moosgummiplatte 24 gehalten, die zur Abschirmung von Fremdlicht mit einer umlaufenden, an dem
die Doppelschlitzblende 22 enthaltenden Rahmen 25
anliegenden Dichtkante 26 versehen ist. Die Moosgummiplatte 24 wird von einer dem Rahmen 25 gegenüberliegenden Stützplatte 27 gehalten, die ihrerseits
gestellseitig gehalten, hier über nicht näher bezeichnete Winkellaschen mit den Seitenwandungen 16
verbunden ist.

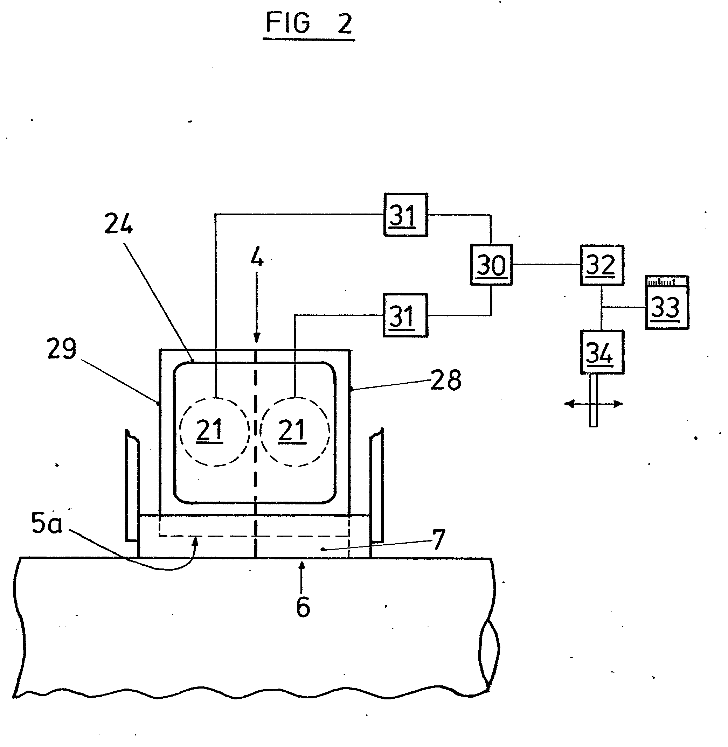

Der vom Fotoempfänger 21 auf Grund des von der Kontaktzone 6 ausgehenden Lichts abgegebene Strom kann
ggf. nach entsprechender Spannungsumwandlung direkt
einem Regler etwa zur Regelung der Temperatur des
Trockners 2 aufgegeben werden. Hierzu ist lediglich
eine entsprechende Eichung der Stromstärke bezüglich der dazugehörigen Abriebfestigkeit vorzunehmen.
Um Schwankungen im Bereich der Lichtquelle, welche
das Meßergebnis verfälschen können, auszuscheiden,
kann mit einem zusätzlichen Referenzstrahl gearbeitet werden, dessen Intensität laufend mit dem eigentlichen Meßstrahl verglichen wird. Hierzu ist der Abschmierer 4, wie am besten aus Figur 2 erkennbar ist, zweispurig ausgebildet, d. h. neben dem eigentlichen Meßprisma 28 ist ein Referenzprisma 29 genau gleicher Geometrie angeordnet, welchem ebenfalls ein Fotoempfänger 21 zugeordnet ist. Die nebeneinander angeordneten Fotoempfänger 21 können dabei mit Hilfe einer gemeinsamen Moosgummiplatte 24 gehalten sein. Die die eigentliche Kontaktzone 6 enthaltende Schutzplatte 7 ist hierbei lediglich im Bereich des Meßprisma 28 vorgesehen. Die dementsprechend freiliegende Hypotenusenfläche 5a des Referenzprismas 29 ist verspiegelt, was etwa durch Aufdampfen einer geeigneten Metallschicht erreicht werden kann. Die hierdurch gebildete Spiegelfläche ist nach innen gewandt, d. h. von der unter dem Abschmierer 4 hindurchgeführten Papierbahn 1 abgewandt. Diese Spiegelfläche bewerkstelligt eine direkte Spiegelung des auftreffenden Lichtstrahls. Gleichzeitig bleibt die direkte Bahnberührung auf das Meßprisma 28 beschränkt, was eine hohe Freizügigkeit hinsichtlich der Einstellung des Abschmierers 4 über der Breite der Bahn 1 ergibt. Die die Beleuchtungseinrichtung 17 bildende Lampe ist beiden Prismen gemeinsam zugeordnet. Jedem Prisma ist eine die gewünschte Fokusierung bewerkstelligende Linse 19 zugeordnet. Die dem Referenzprisma 29 zugeordnete Linse 19 ist dabei gegenüber der dem Meßprisma 28 zugeordneten Linse so weit versetzt, daß die im Bereich beider Prismen reflektierten Strahlen trotz des Höhenunterschieds der reflektierenden Hypotenusenflächen genau nebeneinander sich befinden.

Die Fotoempfänger 21 stellen praktisch Signalwandler dar, welche die Intensität des empfangenen Lichts in eine Stromstärke umsetzen, wobei hier die Differenz zwischen den von den beiden Fotoempfängern 21 abgegebenen Stromstärken ein Maß für die Verschmutzung der Kontaktzone 6 des Meßprismas 28 und dementsprechend ein Maß für die Abriebfestigkeit der auf der Papierbahn 1 sich befindenden Farbe darstellt. Den Fotoempfängern 21 ist daher, wie weiter aus Figur 2 erkennbar ist, eine Differenzbildungseinrichtung 30 nachgeordnet. Hierin kann gleichzeitig eine Verstärkung erfolgen. Der Differenzbildungseinrichtung sind im dargestellten Ausführungsbeispiel Wandler 31 vorgeordnet, welche den vom jeweils zugeordneten Fotoempfänger 21 abgegebenen Strom in eine Spannung umwandeln. Der Ausgang der Differenzbildungseinrichtung 30 liegt an einer Speichereinrichtung 32, welche die eingespeisten Sptzenwerte speichert und hieraus das Meßsignal bildet. Dieses Signal kann einer Anzeigeeinrichtung 33 zugeführt werden, an welcher die gemessene Abriebfestigkeit ablesbar ist. Im dargestellten Ausführungsbeispiel wird dieses Signal gleichzeitig einem Regler 34 zugeführt, mit Hilfe dessen die Temperatur des Trockners 2 direkt oder indirekt geregelt werden soll, was praktisch eine automatisch Trocknerregelung ergibt. Im dargestellten Ausführungsbeispiel wird der Regler 34 dazu benutzt, das in Figur 1 bei 14 angedeutete Gegengewicht, mit welchem die im Bereich der Kontaktzone wirksame Anpreßkraft eingestellt wird, zu verschieben, und zwar derart, daß die Extinktion der Totalreflexion im Bereich der Kontaktzone 6 bei einem gewissen Wert konstant bleibt, d. h. daß die Verschmutzung im Bereich der Kontaktzone 6 unabhängig vom Trockengrad in etwa konstant bleibt, wodurch sichergestellt ist, daß eine sichtbare Beschädigung des Druckbilds auf jedem Fall unterbleibt. Der hierbei sich ergebende Verschiebeweg im Bereich des Gegengewichts 14 kann dann gleichzeitig zur Steuerung der Temperatur des Trockners 2 Verwendung finden.

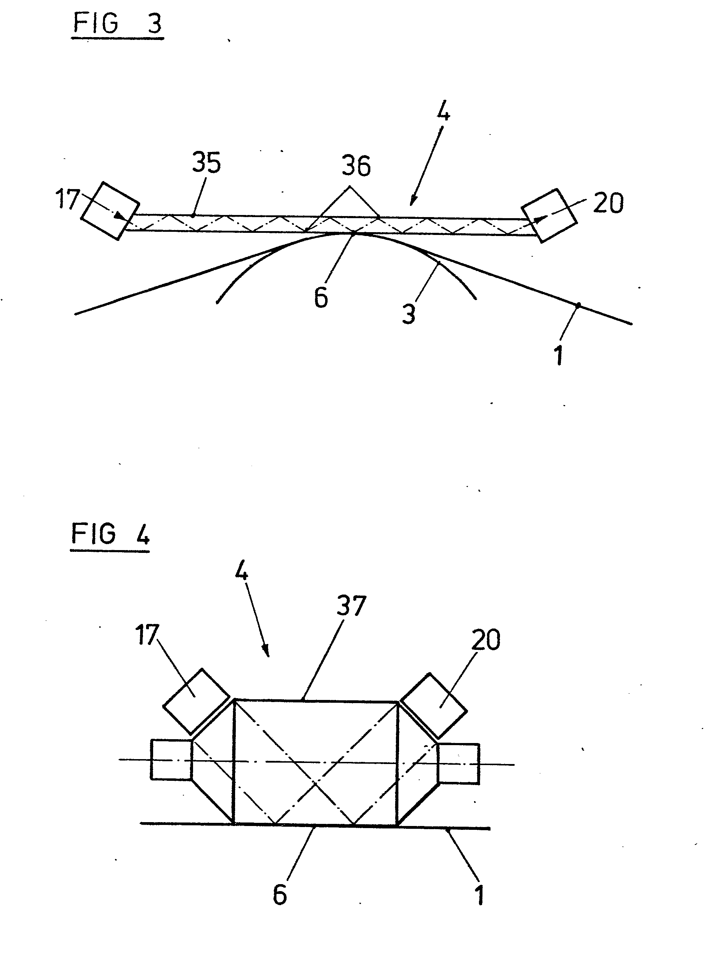

Anstelle eines totalreflektierenden Prismas könnten auch andere optische Bauteile verwendet werden, die auf dem Prinzip der Totalreflexion basieren, wobei diese ebenfalls durch nicht abriebfeste Medien gestört wird.Bei dem in Figur 3 dargestellten Beispiel findet zur Bildung des Abschmierers 4 ein durch ein Saphirblättchen 35 von etwa 0,1 mm Stärke gebildeter Lichtleiter Verwendung. Dieser Lichtleiter ist dabei so zu justieren, daß eine seiner Reflexionsstellen 36 in die Kontaktzone 6 fällt. Die im Bereich der vorderen und hinteren, abgeschrägten Kanten vorgesehene Beleuchtungseinrichtung bzw. Beobachtungseinrichtung sind bei 17 bzw. 20 angedeutet.

Bei dem in Figur 4 dargestellten Beispiel ist der ebenfalls als Ganzes mit 4 bezeichnete Abschmierer praktisch als rotationssymmetrisches Prisma ausgebildet. Hierzu findet eine durchsichtige Walze 37 Verwendung, die drehbar gelagert ist und den Träger 1 im Bereich ihres Umfangs berührt. Die Beleuchtungseinrichtung 17 und die Beobachtungseinrichtung 20 sind im Bereich der Walzenstirnseiten vorgesehen, die zur Bewerkstelligung der gewünschten Neigung der optischen Achsen kegelig angefast sind. Anstelle eines Außenkegels 38 hier verwendeter Art könnte auch ein Innenkegel Verwendung finden. Eine derartige Walze könnte zu Reinigungszwecken vorteilhaft mit zugeordneten Reinigungswalzen in Kontakt gebracht werden. Ferner wäre es denkbar, eine mit dem Träger in Kontakt kommende Zwischenwalze vorzusehen, die ihrerseits praktisch als Zwischenträger mit einem hierauf anliegenden totalreflektierenden, optischen Bauteil zusammenwirkt.

Vorstehend sind zwar einige bevorzugte Ausführungsbeispiele näher erläutert, ohne daß jedoch hiermit eine Beschränkung verbunden sein soll. Dem Fachmann stehen eine Reihe von Möglichkeiten zur Verfügung, um den Grundgedanken der Erfindung an die Verhältnisse des Einzelfalls anzupassen.

A n s p r ü c h e

1. Vorrichtung zur Bestimmung der Abriebfestigkeit von auf einen Träger aufgebrachten Druckfarben, mit einem mit einstellbarer Kraft auf den von einer Gegenkraft, vorzugsweise von einem hiervon leicht umschlungenen Stahlteil, wie einer Stahlwalze gestützten Träger (Papierbahn 1) andrückbaren Abschmierer (4) aus optisch durchsichtigem Material, der einer auf die Berührungsstelle (6) zwischen Träger und Abschmierer fokusierten Beleuchtungseinrichtung (17) ausgesetzt ist und dem eine im Bereich des zurückgeworfenen, aus ihm austretenden Lichts angeordnete Beobachtungseinrichtung (20) zugeordnet ist, dadurch gekennzeichnet, daß die optische Achse der Beleuchtungseinrichtung (17) gegenüber einer zu der die Kontaktzone (6) zwischen Abschmierer (4) und Träger (1) enthaltenden Tangentialebene lotrechten Ebene um jeweils einen Winkel geneigt ist, der zumindest gleich oder vorzugsweise größer als der Grenzwinkel der Totalreflexion für das den Abschmierer bildende Material ist.

2. Vorrichtung nach Anspruch 1, dadurch gekennzeichnet, daß der Abschmierer (4) die Form eines totalreflektierenden Prismas aufweist, das gegenüber dem bewegten Träger (1) festgehalten ist und diesen im Bereich seiner Hypotenusenfläche (5) berührt.

3. Vorrichtung nach Anspruch 2, dadurch gekennzeichnet, daß der Abschmierer (4) einen die Kontaktzone (6) enthaltende, vorzugsweise durch optischen Kitt mit dem übrigen Material verbundene Panzerung (Schutzplatte 7) aus verschleißfestem, denselben Brechungsindex wie das übrige Material aufweisenden Material, vorzugsweise Saphir, aufweist.

4. Vorrichtung nach einem der vorhergehenden Ansprüche 1 bis 3, dadurch gekennzeichnet, daß jeder Eingang der vorzugsweise spiegelbildlich zur Beleuchtungseinrichtung angeordneten Beobachtungseinrichtung (20) mit einer Doppelschlitzblende (22) und einem Filter (23) versehen ist, wobei zumindest der eingangsseitige Schlitz der vorzugsweise durch voneinander distanzierte Platinen gebildeten Doppelschlitzblende (22) einstellbar ist.

5. Vorrichtung nach einem der vorhergehenden Ansprüche 1 bis 4, dadurch gekennzeichnet, daß der vorzugsweise die Beleuchtungseinrichtung (17) bildenden Lampe eine Kühleinrichtung (18), vorzugsweise in Form eines Ventilators, zugeordnet ist.

6. Vorrichtung nach einem der vorhergehenden Ansprüche 1 bis 5, wobei der Abschmierer (4) ein Meßfeld und ein Referenzfeld aufweist, denen im Bereich der Beobachtungseinrichtung (20) jeweils ein vorzugsweise mit einer Steuereinrichtung verbundener Fotoempfänger (21) zugeordnet ist, dadurch gekennzeichnet, daß der Abschmierer (4) ein Meßprisma (28) und ein Referenzprisma (5a) aufweist und daß das Referenzfeld des Referenzprismas als vom Träger (1) abgewandte Spiegelfläche ausgebildet ist und, daß den Fotoempfängern (21) der Beobachtungseinrichtung eine Differenzbildungseinrichtung (30) nachgeordnet ist, der vorzugsweise eine Speichereinrichtung (32) für Spitzenwerte nachgeordnet ist, deren Ausgangssignal einer Regeleinrichtung (34) eingebbar ist, mittels der ein dem Abschmierer (4) vorgeordneter Trockner (2) direkt oder indirekt steuerbar ist, wobei der/die Fotoempfänger (21) vorzugsweise durch mindestens eine gehäuseseitig abgestützte Moosgummiplatte (24) hinterlegt ist/sind, die eine umlaufende, auf der vorzugsweise durch die Doppelschlitzblende (22) gebildeten Unterlage des/der Fotoempfänger (21) lichtdicht aufliegende Dichtkante (26) aufweist.

7. Vorrichtung nach Anspruch 6, dadurch gekennzeichnet, daß die Schutzplatte (7) auf das Meßprisma (28) beschränkt ist und daß beleuchtungsseitig dem Meßprisma (28) und dem fluchtend daneben angeordneten Referenzprisma (29) jeweils eine Linse (19) zugeordnet ist, welche einer gemeinsamen Lichtquelle ausgesetzt sind und in Abhängigkeit von der Dicke der Schutzplatte gegeneinander versetzt sind.

8. Vorrichtung nach einem vorhergehenden Ansprüche 6 oder 7, dadurch gekennzeichnet, daß der Abschmierer (4) mit einer Anfangskraft von etwa 2 N an den zugeordneten Träger anpreßbar ist und daß vorzugsweise mit Hilfe der Regeleinrichtung (34) die auf den Abschmierer (4) wirkende Anpreßkraft so verstellbar ist, daß der Ausgangswert der Differenzbildungseinrichtung (30) bei einem vorgegebenen Wert konstant bleibt.

9. Vorrichtung nach einem der vorhergehenden Ansprüche 1 bis 8, wobei der Abschmierer (4) in einem Rahmen (9) aufgenommen ist, der an einem Schwenkarm (11) mit einstellbarem Schwenkmoment befestigt ist, dadurch gekennzeichnet, daß der Schwenkarm (11) in Achsrichtung verstellbar auf einem Schlitten (12) gelagert ist, der über der Breite des Trägers (1) verstellbar ist.

10. Vorrichtung nach einem der Ansprüche 1 und 3 bis 9, dadurch gekennzeichnet, daß der Abschmierer (4) als drehbare, durchsichtige stirnseitig beleuchtete Walze ausgebildet ist, deren Umfang den Träger (1) berührt und die vorzugsweise kegelig angefaste Stirnseiten aufweist.

11. Vorrichtung nach einem der Ansprüche 1 und 3 bis 9, dadurch gekennzeichnet, daß der Abschmierer (4) als blättchenförmiger, eine Vielzahl von Reflexionsstellen aufweisender Lichtleiter ausgebildet ist, der den Träger (1) im Bereich einer Reflexionsstelle berührt.

FIG 1

# FIG 2

FIG 3

FIG 4

## EUROPÄISCHER RECHERCHENBERICHT

**Europäisches Patentamt**

### EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| Y,D | DE - B - 2 917 519 (FORSCHUNGS-GESELLSCHAFT DRUCKMASCHINEN) <br><br> * das ganze Dokument * | 1,6,9 |
| Y | DE - A - 2 504 199 (AXEL SPRINGER) <br><br> * Ansprüche 1 und 2; Figur * | 1,2,6 |
| A | US - A - 4 003 660 (CHRISTIE) <br><br> * Spalte 10, Zeile 40 bis Spalte 11, Zeile 6; Figur 1 * | 4 |
| A | DE - A - 2 402 435 (BEASLEY FRENCH) <br><br> * Ansprüche 6 und 7 * | 5 |
| A | FR - A - 2 252 926 (VICKERS LTD.) <br><br> * das ganze Dokument * <br><br> & DE - A - 2 456 551 | 6 |
| A | DE - A - 2 944 322 (TEZZELE) <br><br> * Anspruch 3; Figur 7 * | 10 |

**KLASSIFIKATION DER ANMELDUNG (Int. Cl.)**

B 41 F 33/00
G 01 N 33/32

**RECHERCHIERTE SACHGEBIETE (Int. Cl.)**

B 41 F
G 01 N

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| DEN HAAG | 14-04-1982 | LUTZ |

EPA form 1503.1  06.78